Europäisches Patentamt

European Patent Office

Office européen des brevets

(11) Veröffentlichungsnummer: **0 382 054 B1**

(12) **EUROPÄISCHE PATENTSCHRIFT**

(45) Veröffentlichungstag der Patentschrift: **26.04.95**

(21) Anmeldenummer: **90101790.5**

(22) Anmeldetag: **30.01.90**

(51) Int. Cl.6: **C07K 1/00**, C07K 2/00, C09B 67/00

(54) **Chemisch modifizierte Proteine.**

(30) Priorität: **04.02.89 DE 3903362**

(43) Veröffentlichungstag der Anmeldung:
**16.08.90 Patentblatt 90/33**

(45) Bekanntmachung des Hinweises auf die
Patenterteilung:
**26.04.95 Patentblatt 95/17**

(84) Benannte Vertragsstaaten:
**AT BE CH DE DK ES FR GB IT LI NL SE**

(56) Entgegenhaltungen:
**EP-A- 0 120 469**
**EP-A- 0 290 306**

(73) Patentinhaber: **BASF Aktiengesellschaft**
**Carl-Bosch-Strasse 38**
**D-67063 Ludwigshafen (DE)**

(72) Erfinder: **Brueckmann, Ralf, Dr.**
**In den Bannzaeunen 17**
**D-6701 Goennheim (DE)**
Erfinder: **Dix, Johannes Peter, Dr.**
**Am Haengel 5**
**W-6719 Weisenheim (DE)**
Erfinder: **Herrmann, Manfred, Dr.**
**Parkstrasse 23**
**D-6700 Ludwigshafen (DE)**
Erfinder: **Leiter, Herbert, Dr.**
**Salierstrasse 11**
**D-6701 Maxdorf (DE)**
Erfinder: **Zimmermann, Norbert**
**Jahnstrasse 6**
**D-6701 Waldsee (DE)**

EP 0 382 054 B1

**Beschreibung**

Die vorliegende Erfindung betrifft neue chemisch modifizierte Proteine, die durch Umsetzung von Casein mit 0,5 bis 50 Gew.% einer nicht aromatischen Mono- oder Disulfonsäure, die in ihrem organischen Rest eine oder mehrere mit Nucleophilen reagierende Gruppen oder Gruppierungen enthält, in wäßrigem Medium bei einem pH-Wert von 6 bis 14 erhältlich sind, ein Verfahren zu ihrer Herstellung, ihre Verwendung als Dispergiermittel in Farbmittelzubereitungen sowie diese Zubereitungen.

Als Dispergiermittel für Farbmittel, insbesondere für Küpen- und Dispersionsfarbstoffe, werden üblicherweise Ligninsulfonate, Naphthalinsulfonsäure-Formaldehyd-Kondensate oder Sulfonate von Phenol-Formaldehyd-Kondensaten verwendet. Die genannten Hilfsstoffe sind nur zu einem kleinen Teil in Kläranlagen biologisch abbaubar oder eliminierbar und können somit zur Belastung der Oberflächengewässer beitragen.

In der DE-A 27 34 204 werden Copolymerisate aus Styrol und Acrylsäure im Gewichtsverhältnis von 50:50 bis 70:30 und aus Styrol, Acrylsäure und Maleinsäureanhydrid im Gewichtsverhältnis von 50:40:10 bis 70:24:6 in Form von wasserlöslichen Salzen als Dispergiermittel für Farbstoffe beschrieben. Diese Copolymerisate sind wesentlich weniger umweltbelastend als die oben genannten Dispergiermittel und zeigen auch eine akzeptable Dispergierwirkung. Trotzdem sind aber die dispergierenden Eigenschaften noch weiter verbesserungsbedürftig.

Die Literatur kennt Beispiele für die Verwendung von natürlich vorkommenden Eiweißstoffen als Schutzkolloide und Dispergiermittel. So beschreibt Athey in Tappi, Bd. 58, Nr. 10, S. 55-61 (1975) unter anderem Casein als Dispergiermittel für Pigmente bei der Papierbeschichtung. Die EP-B 018 947 betrifft Casein in Form seines Ammoniumsalzes als Emulgiermittel beim Färben von Polyestergewebe. Die natürlich vorkommenden Proteine und ihre Salze sind biologisch nahezu vollständig abbaubar oder eliminierbar, jedoch lassen ihre dispergierenden Eigenschaften im Hinblick auf Farbmittelzubereitungen, vor allem bezüglich der Dispersionsstabilität, zu wünschen übrig.

Aus der EP-A 290 306 ist ein Verfahren zur Herstellung von Kunststoffmaterialien bekannt, bei dem man Casein durch Erhitzen in Gegenwart starker Säuren wie Salzsäure partiell hydrolysiert und anschließend die erhaltenen Oligomeren mit einer Monohalogencarbonsäure umsetzt. Die so hergestellten Materialien eignen sich als Beschichtungsmittel auf beispielsweise Möbeln oder Schmuckgegenständen.

Der vorliegenden Erfindung lag daher die Aufgabe zugrunde, Dispergiermittel bereitzustellen, die bei nahezu vollständiger biologischer Abbaubarkeit oder Eliminierbarkeit in ihren dispergierenden Eigenschaften den hohen Anforderungen für Farbmittelzubereitungen gerecht werden.

Demgemäß wurden die eingangs definierten chemisch modifizierten Proteine gefunden.

Das eingesetzte Protein Casein (Milcheiweiß) kommt in tierischen Organismen vor.

Die Umsetzung von Casein mit 0,5 bis 50 Gew.%, vorzugsweise 1 bis 20 Gew.% einer nicht aromatischen Mono- oder Disulfonsäure, die in ihrem organischen Rest eine oder mehrere mit Nucleophilen reagierende Gruppen oder Gruppierungen enthält, erfolgt in wäßriger Lösung oder Suspension bei einem pH-Wert von 6 bis 14, vorzugsweise 7 bis 12. Geeignete Gruppen oder Gruppierungen, die zu einem Angriff auf nucleophile Reaktionszentren in den Eiweißstoffen führen können, sind beispielsweise mit Hydroxylgruppen, Sulfatogruppen, Chlor oder Brom substituierte Alkylreste, vorzugsweise $C_1$-$C_4$-Alkylreste, sowie die Epoxygruppe und die olefinisch ungesättigte Doppelbindung, vorzugsweise in $\alpha,\beta$-Position. Als Beispiele für derartige Mono- oder Disulfonsäuren lassen sich Hydroxymethansulfonsäure, Chlormethansulfonsäure, Brommethansulfonsäure, Vinylsulfonsäure, 2-Chlorethansulfonsäure, 2-Bromethansulfonsäure, 2-Sulfatoethansulfonsäure, 3-Chlor-2-hydroxypropan-sulfonsäure, 3-Brom-2-hydroxypropan-1-sulfonsäure, 2,3-Epoxypropan-1-sulfonsäure und 1,2-Dihydroxyethan-1,2-disulfonsäure nennen. Die Sulfonsäuren werden vorzugsweise als Alkalimetall-, Erdalkalimetall-, Ammonium- oder Aminsalze eingesetzt.

Vor der Umsetzung mit den Sulfonsäuren wird der Eiweißstoff üblicherweise mit Hilfe von Basen gelöst oder zumindest angelöst und der gewünschte pH-Bereich wird eingestellt. Als Basen können Alkalimetall- oder Erdalkalimetalloxide oder -hydroxide, insbesondere Natrium- oder Kaliumhydroxid, Ammoniak oder Amine, insbesondere Alkanolamine wie Mono-, Di- oder Triethanolamin, eingesetzt werden. Auch Mischungen der genannten Basen können verwendet werden.

Man arbeitet bei der Umsetzung von Casein mit den Sulfonsäuren in der Regel bei Temperaturen zwischen 20 und 100°C, vorzugsweise zwischen 50 und 95°C; die Umsetzung kann jedoch auch bei Temperaturen über 100°C unter Druck durchgeführt werden.

Die erfindungsgemäßen chemisch modifizierten Proteine können in Form ihren wäßrigen Lösungen, die nach ihrer Herstellung in der Regel auf einen pH-Wert zwischen 7 bis 10 eingestellt werden und eine niedrige Viskosität von üblicherweise 10 bis 200 mPa.s, in wenigen Fällen bis 2.500 mPa.s, bei 20°C aufweisen, oder nach einer der üblichen Aufarbeitungsmethoden, beispielsweise Sprühtrocknung, Gefriertrocknung oder Eindampfen, in fester Form als Dispergiermittel in Farbmittelzubereitungen verwendet

2

werden.

Die erfindungsgemäßen Farbmittelzubereitungen sind entweder wasserfreie, meist in Pulverform vorliegende Feststoffe oder stabile wäßrige Dispersionen. Unter Farbmitteln sind Farbstoffe, beispielsweise Textilfarbstoffe, und Pigmente zu verstehen. Bei den Farbstoffen handelt es sich in der Regel um in Wasser schwerlösliche bis unlösliche Substanzen. Insbesondere betrifft die vorliegende Erfindung Dispersionsfarbstoffe, Küpenfarbstoffe und optische Aufheller. Die genannten Farbstofftypen umfassen vor allem Vertreter aus den Klassen der Azo-, Anthrachinon- und Chinophthalon-Farbstoffe.

Die erfindungsgemäßen Farbmittelzubereitungen enthalten im Falle der festen Zubereitungen 0,001 bis 10 Gew.-Teile, vorzugsweise 0,1 bis 2,5 Gew.-Teile, insbesondere 0,7 bis 2,2 Gew.-Teile, und im Falle der flüssigen Zubereitungen 0,001 bis 2 Gew.-Teile, vorzugsweise 0,05 bis 1,5 Gew.-Teile, insbesondere 0,1 bis 0,6 Gew.-Teile der chemisch modifizierten Proteine pro Gew.-Teil des Farbmittels.

Besonders vorteilhafte Farbmittelzubereitungen sind solche, die neben den erfindungsgemäßen Proteinderivaten Copolymerisate aus olefinisch ungesättigten Carbonsäuren und/oder Carbonsäureanhydriden einerseits und wasserunlöslichen Monomeren andererseits in Form von wasserlöslichen Salzen als zusätzliches Dispergiermittel enthalten. Diese Copolymerisate sind als Dispergiermittel an sich bekannt, beispielsweise aus der DE-A 27 34 204. Ihre Dispergierwirkung beruht auf dem Prinzip, daß sie im Polymermolekül hydrophile Zentren wie Carboxylatgruppen neben hydrophoben Polymerketten aufweisen.

Besonders gut geeignete Copolymerisate dieser Art sind z.B. aus

- 30 bis 50 Gew.% Acrylsäure und
  50 bis 70 Gew.% Styrol oder
- 24 bis 40 Gew.% Acrylsäure,
  6 bis 10 Gew.% Maleinsäureanhydrid und
  50 bis 70 Gew.% Styrol

aufgebaut. Diese nach den üblichen Methoden der radikalischen Polymerisation erhältlichen Copolymerisate werden in einer vollständig oder nahezu vollständig mit Alkalimetallhydroxiden, beispielsweise Natrium-, Kalium- oder Lithiumhydroxid, Ammoniak oder Alkanolaminen, beispielsweise Triethanolamin, Tri-n-propanolamin, Triisopropanolamin oder Tetra(hydroxypropyl)ethylendiamin, neutralisierten Form in wäßriger Lösung eingesetzt.

Die erfindungsgemäßen Farbmittelzubereitungen können diese wasserlöslichen Salze der Copolymerisate oder Gemische hiervon im Falle der festen Zubereitungen in einer Menge von bis zu 8 Gew.-Teilen, vorzugsweise bis zu 2 Gew.-Teilen, insbesondere bis zu 1,1 Gew.-Teilen, und im Falle der flüssigen Zubereitungen bis zu 1,5 Gew.-Teilen, vorzugsweise bis zu 0,5 Gew.-Teilen, insbesondere bis zu 0,15 Gew.-Teilen, pro Gew.-Teil des Farbmittels enthalten.

Die erfindungsgemäßen flüssigen Farbmittelzubereitungen enthalten neben dem Farbmittel und den Dispergiermitteln in der Regel 20 bis 100 Gew.%, bezogen auf die Gesamtmenge an Feststoff der wäßrigen Dispersion, Wasser oder ein Gemisch aus Wasser und Wasserrückhaltemitteln. Als Wasserrückhaltemittel können Ethylenglykol, Diethylenglykol, Triethylenglykol, Propylenglykol, Dipropylenglykol, Diethylenglykolmonobutylether, Glycerin, Sorbit, Dextrin, 2-Butin-1,4-diol oder 2-Methoxypropan-1-ol verwendet werden. Wasserrückhaltemittel verhindern das schnelle Antrocknen von dünnen Filmen der Farbmitteldispersion.

Außerdem können in den erfindungsgemäßen Farbmittelzubereitungen in geringen Mengen, etwa bis 20 Gew.%, bezogen auf die Gesamtfarbmittelzubereitung, folgende weitere übliche Hilfsmittel mit anwesend sein:

- Konservierungsmittel (Biozide) zur Verhinderung des Bakterien- und Pilzbefalls, beispielsweise p-Chlor-m-kresol, 1,2-Benzisothiazolin-3-on und Chloressigsäureamid
- pH-Wert-regulierende Substanzen, insbesondere Puffermischungen, enthaltend beispielsweise Alkalimetallhydroxide, Mono-, Di- und Triethanolamin, Schwefelsäure, Salzsäure, Phosphorsäure, Essigsäure und die Alkalimetall- und Ammoniumsalze der genannten Säuren
- Antischaummittel zur Unterdrückung des Schäumens beim Mahlen der Farbmittelzubereitung und beim Färben mit ihr, beispielsweise Fettalkohole, Fettalkoholalkoxylate, Alkylester von Carbonsäuren und Phosphorsäure sowie siliconhaltige Entschäumer
- Viskositätsregler zur Verbesserung des rheologischen Verhaltens von Farbmittelzubereitungen, beispielsweise zur Viskositätsherabsetzung Harnstoff, N-Methylacetamid, Toluolsulfonate, Cumolsulfonate und Naphthalinsulfonate und zur Viskositätserhöhung Cellulose-Derivate und Polyacrylate
- Netzmittel zur Verbesserung der Redispergierbarkeit von pulverförmigen Farbmitteln sowie Farbmittelantrocknungen, beispielsweise Sulfobernsteinsäurealkylester, Dialkylsulfimide, Phosphorsäurealkylester und Fettalkoholalkoxylate
- Entstaubungsmittel zur Vermeidung der Staubbildung beim Umgang mit pulverförmigen Farbmittelzubereitungen, insbesondere Mischungen aus einer Ölkomponente und einem geeigneten Emulgatorsy-

3

stem

Die Herstellung der erfindungsgemäßen Farbmittelzubereitungen erfolgt in an sich bekannter Weise durch Mahlen und Dispergieren des Farbmittels, in der Regel in wäßrigem Medium, beispielsweise in Knetern, in Kugelmühlen, in Sandmühlen, in Perlmühlen oder in Attritoren. Hierbei kann das Farbmittel in Form eines trockenen Pulvers oder vorzugsweise in Form des bei der Herstellung des Farbmittels anfallenden wasserhaltigen Preßkuchens eingesetzt werden. Zur Herstellung der erfindungsgemäßen pulverförmigen Farbmittelzubereitungen wird die erhaltene wäßrige Farbmitteldispersion in der Regel in bekannter Weise sprühgetrocknet, beispielsweise bei Temperaturen zwischen 60 und 160 °C.

Die erfindungsgemäßen Farbmittelzubereitungen sind für alle Färbe- und Druckverfahren geeignet.

Die erfindungsgemäßen chemisch modifizierten Proteine und auch die in Kombination mit ihnen anzuwendenden Copolymerisate aus beispielweise Styrol, Acrylsäure und Maleinsäureanhydrid sind nahezu vollständig biologisch abbaubar oder eliminierbar und somit nicht umweltbelastend. Der biologische Abbaugrad im Zahn-Wellens-Test, definiert durch die Bestimmung des chemischen Sauerstoffbedarfs während des Abbauvorgangs, ist für beide Substanzklassen in der Regel größer als 90 %. Die üblicherweise als Dispergiermittel für Farbstoffzubereitungen benutzten Ligninsulfonate, Naphthalinsulfonsäure-Formaldehyd-Kondensate und Sulfonate von Phenol-Formaldehyd-Kondensaten zeigen dagegen meist Abbaugrade von unter 40 %.

Die erfindungsgemäßen chemisch modifizierten Proteine weisen eine hohe Dispergierwirkung auf. Die mit ihnen hergestellten Farbmitteldispersionen sind sehr feinteilig und zugleich stabil. Die erfindungsgemäßen Farbmittelzubereitungen haben eine hohe Wärmestabilität, d.h. eine hohe Beständigkeit der Farbmitteldispersionen unter Färbebedingungen in den heißen Färbeflotten. Außerdem zeigen sie eine hohe Redispergierbarkeit bei pulverförmigen Zubereitungen und bei Farbmittelantrocknungen auf.

Als weiterer Vorteil der erfindungsgemäßen chemisch modifizierten Proteine ist ihre helle Farbe anzuführen. Somit besteht bei ihrer Verwendung als Dispergiermittel in Farbmittelzubereitungen nicht mehr die Gefahr, daß beim Färbevorgang von Textilien Begleitgewebe angeschmutzt wird. Dies ist wichtig bei brillianten Farbtönen und vor allem beim Behandeln von Textilgewebe mit optischen Aufhellern.

Die Kombination der erfindungsgemäßen chemisch modifizierten Proteine mit beispielsweise den aus der DE-A 27 34 204 bekannten Copolymerisaten aus Styrol, Acrylsäure und gegebenenfalls Maleinsäureanhydrid bringt nochmals eine deutliche Verbesserung des Dispergiervermögens. Dies zeigt sich vor allem an der größeren Wärmebelastbarkeit und der längeren Lagerfähigkeit dieser Zubereitungen. Als zusätzlicher Vorteil ist hier die geringe Migrationsneigung von Farbstoffen bei Kontinue-Färbeverfahren zu nennen, d.h. die Färbungen weisen eine höhere Egalität auf.

Die in den Beispielen verwendeten Prozentangaben beziehen sich - wenn nichts anderes angegeben ist - auf das Gewicht. Die Viskositäten wurden in einem Rotationsviskosimeter (Haake, Rotavisco) bei 20°C gemessen.

Der biologische Abbaugrad wurde nach dem Zahn-Wellens-Test gemäß DIN 38412 Teil 25 ermittelt. Dieser Test bedient sich eines belebten Schlammes mit einer Mischung verschiedener Mikroorganismen und mineralischer Nährstoffe zum biologischen Abbau der Prüfsubstanz. Die wäßrige Lösung dieser drei Bestandteile wurde während einer bestimmten Zeit (bis zu 28 Tagen) bei einer konstanten Temperatur von ca. 22°C unter weitgehendem Lichtausschluß belüftet. Die Abnahme der Menge der Prüfsubstanz wurde durch Bestimmung des chemischen Sauerstoffbedarfs ermittelt.

Handelsübliches Naphthalinsulfonsäure-Formaldehyd-Kondensat zeigte bei dieser Meßmethode einen Abbaugrad von 38 % und Ligninsulfonat einen Wert von 33 %.

Herstellungsbeispiele

Beispiel 1

Umsetzung von Casein mit Natriumhydroxymethansulfonat in natronalkalischer Lösung

In eine Mischung aus 1 500 g Wasser und 54 g 50 %iger Natronlauge wurden bei 60°C 300 g Casein portionsweise eingetragen und durch einstündiges Rühren bei dieser Temperatur weitgehend gelöst. Zu dieser Lösung, die einen pH-Wert von 10,5 hatte, wurden bei der gleichen Temperatur 182 g einer 33 %igen wäßrigen Natriumhydroxymethansulfonat-Lösung (entsprechend 20,0 % wasserfreies Reagenz, bezogen auf Casein) innerhalb von 30 Minuten zugetropft. Danach wurde die Reaktionsmischung 3 Stunden bei 90°C gerührt. Die so erhaltene Lösung hatte einen pH-Wert von 9,5 und eine Viskosität von 21 mPa.s. Der biologische Abbaugrad betrug 93 %.

4

Beispiel 2

Umsetzung von Casein mit Natriumvinylsulfonat in natronalkalischer Lösung

In eine Mischung aus 3 500 g Wasser und 252 g 50 %iger Natronlauge wurden bei 60°C portionsweise 1 300 g Casein eingetragen und durch einstündiges Rühren bei dieser Temperatur weitgehend gelöst. Zu dieser Lösung, die einen pH-Wert von 8,5 hatte, wurden bei der gleichen Temperatur 520 g einer 25 %igen wäßrigen Natriumvinylsulfonat-Lösung (entsprechend 10,0 % wasserfreies Reagenz, bezogen auf Casein) innerhalb von 1 Stunde zugetropft. Anschließend wurde die Reaktionsmischung 8 Stunden bei 90°C gerührt. Nach Abkühlung auf Raumtemperatur wurde durch Zugabe von 45 g 36 %iger Salzsäure ein pH-Wert von 9 eingestellt. Die so erhaltene klare Lösung hatte eine Viskosität von 24 mPa.s. Der biologische Abbaugrad betrug 90 %.

Beispiel 3

Umsetzung von Casein mit Natrium-3-chlor-2-hydroxypropan-1-sulfonat in natronalkalischer Lösung

In eine Mischung aus 1 000 g Wasser und 36 g 50 %iger Natronlauge wurden bei 60°C portionsweise 200 g Casein eingetragen und durch einstündiges Rühren bei dieser Temperatur weitgehend gelöst. Mit 50 %iger Natronlauge wurde anschließend ein pH-Wert von 10 eingestellt. Zu dieser Lösung wurden bei 60°C 10 g Natrium-3-chlor-2-hydroxypropan-1-sulfonat (entsprechend 5,0 %, bezogen auf Casein) gegeben. Danach wurde die Reaktionsmischung 2 Stunden bei 80°C gerührt. Die so erhaltene klare Lösung hatte einen pH-Wert von 11 und eine Viskosität von 15 mPa.s.

Beispiel 4

Umsetzung von Casein mit Dinatrium-1,2-dihydroxyethan-1,2-disulfonat in natronalkanischer Lösung

In eine Mischung aus 1.000 g Wasser und 36 g 50 %iger Natronlauge wurden bei 60°C portionsweise 200 g Casein eingetragen und durch einstündiges Rühren bei dieser Temperatur weitgehend gelöst. Zu dieser Lösung, die einen pH-Wert von 8,7 hatte, wurden bei der gleichen Temperatur 153 g einer 16 %igen wäßrigen Dinatrium-1,2-dihydroxyethan-1,2-disulfonat-Lösung (entsprechend 12,2 % wasserfreies Reagenz, bezogen auf Casein) innerhalb von 0,7 Stunden zugetropft. Anschließend wurde die Reaktionsmischung 3 Stunden bei 90°C gerührt. Nach Abkühlung auf Raumtemperatur wurde mit wenig 50 %iger Natronlauge ein pH-Wert von 8 eingestellt. Die erhaltene klare Lösung hatte eine Viskosität von 2 100 mPa.s. Der biologische Abbaugrad betrug 94 %.

Beispiele 5 bis 9

Copolymerisate aus Styrol, Acrylsäure und Maleinsäureanhydrid

Gemäß der DE-A 27 34 204 wurden Copolymerisate aus Styrol und Acrylsäure (Beispiel 5) und Styrol, Acrylsäure und Maleinsäureanhydrid (Beispiele 6 bis 9) hergestellt. Das Terpolymer hatte einen biologischen Abbaugrad von 95 %. Das Copolymerisat wurde jeweils durch Umsetzung in wäßrigem Medium bei ca. 60°C mit der in Tabelle 1 genannte Base in ein wasserlösliches Salz übergeführt. Der Neutralisationsgrad betrug jeweils 100 mol%. Diese Lösungen mit einem Feststoffgehalt zwischen 20 und 40 % wurden als zusätzliches Dispergiermittel für Farbmittelzubereitungen verwendet.

Tabelle 1

| Neutralisierte Copolymerisate | | | | |
|---|---|---|---|---|
| Beispiel Nr. | Zusammensetzung (%) | | | Base |
| | Styrol | Acrylsäure | Maleinsäureanhyrid | |
| 5 | 70 | 30 | | Triethanolamin |
| 6 | 60 | 30 | 10 | Triethanolamin |
| 7 | 60 | 30 | 10 | Tetra(hydroxypropyl)ethylendiamin |
| 8 | 60 | 30 | 10 | Natriumhydroxid |
| 9 | 60 | 30 | 10 | Lithiumhydroxid |

Anwendungsbeispiele

Die Feinverteilung in den Farbmitteldispersionen wurde durch den Schleuderwert nach Richter und Vescia, Melliand Textilberichte, 1965, Heft 6, S. 621-625, charakterisiert. Die Zahlenwerte entsprechen dem Farbmittelanteil in %, der beim Zentrifugieren bei den Umdrehungszahlen von 1000, 2000 und 4000 pro Minute nach 5 Minuten sedimentiert (1. bis 3. Wert) und der zum Schluß noch in der Dispersion verbleibt (4. Wert). Farbmittel mit kleinen Sedimentationswerten und hohem Endwert sind besonders feinteilig.

Die Schleuderwerte von Vergleichsversuchen mit handelsüblichem Naphthalinsulfonsäure-Formaldehyd-Kondensat und Ligninsulfonat sind in den Tabellen 2 und 3 mit aufgeführt (Beispiele 23 bis 28 und 38 bis 42).

Beispiele 10 bis 22 und Vergleichsbeispiele 23 bis 28.

Flüssige Farbmittelzubereitungen

Die jeweils in Tabelle 2 genannten Mengen, jeweils bezogen auf die wasserfreie Substanz, wenn nichts anderes angegeben ist, an Farbmittel, das in Form eines wasserfeuchten Preßkuchens eingesetzt wurde, Dispergiermitteln und Hilfsmitteln wurden unter intensivem Rühren mit Wasser angeteigt, wobei die Gesamtmenge der Mischung jeweils 1000 g betrug. Anschließend wurde die Mischung nach den in Tabelle 2 genannten Bedingungen (Perlmahlung: Dauer; Sandmahlung: Anzahl der Mahlvorgänge bei einer Durchlaufzeit durch die Mühle von ca. 45 Minuten) bis zur Erreichung einer guten Feinverteilung, charakterisiert durch die Schleuderwerte, gemahlen. Der pH-Wert der Dispersionen wurde auf den jeweils angegebenen Wert durch Zugabe von Essigsäure oder Natronlauge eingestellt und während des Mahlens gehalten.

Beispiele 29 bis 37 und Vergleichsbeispiele 38 bis 42

Feste Farbmittelzubereitungen

Die jeweils in Tabelle 3 genannten Mengen, jeweils bezogen auf die wasserfreie Substanz, wenn nichts anderes angegeben ist, an Farbmittel, das in Form eines wasserfeuchten Preßkuchens eingesetzt wurde, Dispergiermitteln und gegebenenfalls Hilfsmitteln wurden unter intensivem Rühren mit ca. 25 % (bei Beispiel 29-31 und 38), ca. 60 % (bei Beispiel 34-37, 40 und 41) oder 100 % (bei Beispiel 32, 33, 39 und 42) der insgesamt benötigten Menge an Wasser angeteigt, wobei sich die benötigte Menge an Wasser nach dem Feststoffgehalt der Dispersion vor der Sprühtrocknung errechnete. Anschließend wurde die Mischung bei einem pH-Wert von 8 bis 9 im Falle der Farbmittel J und K oder 10 bis 11 im Falle der Farbmittel F, G und H nach den in Tabelle 3 genannten Bedingungen (Perlmahlung: Dauer; Sandmahlung: Anzahl der Mahlvorgänge bei einer Durchlaufzeit durch die Mühle von ca. 45 Minuten) bis zur Erreichung einer guten Feinverteilung gemahlen.

Danach wurden gegebenenfalls weitere Mengen an Dispergiermittel und das restliche Wasser zugegeben. Diese Dispersionen wurden jeweils bei der in Tabelle 3 angegebenen Temperatur sprühgetrocknet. Die prozentuale Zusammensetzung der Farbmittelzubereitung ist in Tabelle 3 angegeben. Die Schleuderwerte wurden nach Wiedereinrühren der erhaltenen pulverförmigen Zubereitungen in Wasser bestimmt.

## Tabelle 2

### Flüssige Farbmittelzubereitungen

| Beispiel Nr. | Farbmittel | Menge [g] | Dispergiermittel aus Beispiel Nr. | Menge [g] | Hilfsmittel | Menge [g] | Mahldauer und -art [h] | | pH-Wert | Schleuder- wert |
|---|---|---|---|---|---|---|---|---|---|---|
| 10 | A | 220 | 1 | 80 | L | 150 | 5 | P | 8-8,5 | 6/13/33/48 |
| | | | | | M | 10 | | | | |
| 11 | A | 220 | 2 | 80 | L | 150 | 6 | P | 8-8,5 | 4/16/28/52 |
| | | | | | M | 10 | | | | |
| 12 | B | 250 | 2 | 130 | L | 50 | 7 | P | 8-8,5 | 2/21/19/58 |
| | | | | | M | 10 | | | | |
| | | | | | N | 50 | | | | |
| 13 | B | 250 | 1 | 80 | L | 50 | 7 | P | 8-8,5 | 8/17/22/53 |
| | | | | | M | 10 | | | | |
| | | | | | N | 50 | | | | |
| 14 | C | 170 | 2 | 80 | L | 100 | 6 | P | 8-8,5 | 7/12/20/61 |
| | | | | | M | 10 | | | | |
| 15 | C | 170 | 1 | 80 | L | 100 | 5 | P | 8-8,5 | 9/16/24/51 |
| | | | | | M | 10 | | | | |

EP 0 382 054 B1

Tabelle 2 (Forts.)

Flüssige Farbmittelzubereitungen

| Beispiel Nr. | Farbmittel | Menge [g] | Dispergiermittel aus Beispiel Nr. | Menge[g] | Hilfsmittel | Menge [g] | Mahldauer und -art [h] | | pH-Wert | Schleuder- wert |
|---|---|---|---|---|---|---|---|---|---|---|
| 16 | D | 200 | 2 | 80 | M | 10 | 2 | P | 8-8,5 | 3/6/22/69 |
| | | | | | N | 100 | | | | |
| | | | | | Q | 50 | | | | |
| 17 | D | 200 | 1 | 80 | M | 10 | 2 | P | 8-8,5 | 1/9/25/65 |
| | | | | | N | 100 | | | | |
| | | | | | Q | 50 | | | | |
| 18 | D | 200 | 4 | 80 | M | 10 | 2 | P | 8-8,5 | 5/12/19/64 |
| | | | | | N | 100 | | | | |
| | | | | | Q | 50 | | | | |

EP 0 382 054 B1

Tabelle 2 (Forts.)

Flüssige Farbmittelzubereitungen

| Beispiel Nr. | Farbmittel | Menge [g] | Dispergiermittel aus Beispiel Nr. | Menge[g] | Hilfsmittel | Menge [g] | Mahldauer und -art [h] | | pH-Wert | Schleuder- wert |
|---|---|---|---|---|---|---|---|---|---|---|
| 19 | E | 250 | 1 | 80 | R | 150 | 16 | P | 8,5-9 | 13/13/31/33 |
| | | | | | T | 150 | | | | |
| 20 | E | 250 | 2 | 80 | R | 150 | 16 | P | 8,5-9 | 12/23/32/33 |
| | | | | | T | 150 | | | | |

EP 0 382 054 B1

Tabelle 2 (Forts.)

Flüssige Farbmittelzubereitungen

| Beispiel Nr. | Farbmittel | Menge [g] | Dispergiermittel aus Beispiel Nr. | Menge[g] | Hilfsmittel | Menge [g] | Mahldauer und -art [h] | | pH-Wert | Schleuder- wert |
|---|---|---|---|---|---|---|---|---|---|---|
| 21 | H | 230 | 1 | 70 | L | 200 | 4xS | | 10-11 | 4/22/30/44 |
| | | | | | M | 10 | | | | |
| 22 | H | 230 | 4 | 50 | L | 200 | 6 | P | 10-11 | 2/22/33/43 |
| | | | | | M | 10 | | | | |

EP 0 382 054 B1

Tabelle 2 (Forts.)

Flüssige Farbmittelzubereitungen

| Vergleichs-beispiel Nr. | Farbmittel | Menge [g] | Dispergiermittel | Menge[g] | Hilfsmittel | Menge [g] | Mahldauer und -art [h] | | pH-Wert | Schleuder-wert |
|---|---|---|---|---|---|---|---|---|---|---|
| 23 | A | 220 | Y | 120 | L | 150 | 5 | P | 8-8,5 | 2/10/31/52 |
|  |  |  |  |  | M | 10 |  |  |  |  |
| 24 | C | 170 | Y | 80 | L | 100 | 6 | P | 8-8,5 | 8/16/29/47 |
|  |  |  |  |  | M | 10 |  |  |  |  |
| 25 | D | 200 | Y | 100 | M | 10 | 2 | P | 8-8,5 | 6/17/30/47 |
|  |  |  |  |  | N | 100 |  |  |  |  |
|  |  |  |  |  | Q | 50 |  |  |  |  |
| 26 | F | 190 | X | 40 | L | 200 | 6 | P | 10-11 | 2/5/27/66 |
|  |  |  |  |  | M | 10 |  |  |  |  |
| 27 | G | 210 | Y | 50 | M | 10 | 5xS |  | 10-11 | 2/7/34/57 |
|  |  |  |  |  | N | 180 |  |  |  |  |
| 28 | H | 230 | Y | 110 | L | 200 | 6 | P | 10-11 | 5/15/33/47 |
|  |  |  |  |  | M | 10 |  |  |  |  |

EP 0 382 054 B1

Tabelle 3

Feste Farbmittelzubereitungen

| Beispiel Nr. | Farbmittel | | | Dispergiermittel aus Beispiel Nr./Hilfmittel | | | | | Mahldauer und -art | Feststoff-gehalt der Dispersion vor Sprüh-trocknung | Tempera-tur bei Sprüh-trocknung | Schleuder-wert |
| | | | | | Menge vor / nach der Mahlung | | zus. | | | | | |
| | | Menge | | | | | | | | | | |
| | | [g] | [%] | | [g] | [g] | [g] | [%] | [h] | [%] | [C°] | |
| 29 | J | 300 | 40 | 1 | 200 | 250 | 450 | 60 | 4xS | 25 | 90 | 4/14/32/60 |
| 30 | J | 300 | 40 | 2 | 200 | 250 | 450 | 60 | 3xS | 25 | 100 | 5/12/24/59 |
| 31 | J | 300 | 40 | 2 | 160 | 200 | 360 | 48 | 5xS | 25 | 110 | 4/11/18/67 |
| | | | | 5 | 40 | 50 | 90 | 12 | | | | |
| 32 | K | 200 | 32 | 1 | 150 | 62,5 | 212,5 | 34 | 3 P | 29 | 100 | 5/9/20/66 |
| | | | | 5 | 150 | 62,5 | 212,5 | 34 | | | | |
| 33 | K | 200 | 32 | 2 | 300 | 125 | 425 | 68 | 3 P | 29 | 100 | 9/17/21/53 |
| 34 | F | 190 | 36,5 | 2 | 36 | 162 | 198 | 38 | 4 P | 17 | 130 | 2/5/11/82 |
| | | | | 8 | 24 | 108 | 132 | 25,5 | | | | |
| 35 | F | 190 | 36,5 | 2 | 60 | 270 | 330 | 63,5 | 6 P | 17 | 130 | 2/6/8/84 |

EP 0 382 054 B1

Tabelle 3 (Forts.)

Feste Farbmittelzubereitungen

| Beispiel Nr. | Farbmittel | | | Dispergiermittel aus Beispiel Nr./Hilfmittel | | | | | Mahldauer und -art | Feststoff-gehalt der Dispersion vor Sprüh-trocknung | Tempera-tur bei Sprüh-trocknung | Schleuder-wert |
|---|---|---|---|---|---|---|---|---|---|---|---|---|
| | | Menge | | | Menge vor / nach der Mahlung | | zus. | | | | | |
| | | [g] | [%] | | [g] | [g] | [g] | [%] | [h] | [%] | [C°] | |
| 36 | G | 265 | 40,6 | 2 | 135 | 96 | 231 | 35,3 | 4 P | 32 | 130 | 3/8/10/79 |
| | | | | 8 | 90 | 64 | 154 | 23,6 | | | | |
| | | | | U | 3,5 | – | 3,5 | 0,5 | | | | |
| 37 | G | 265 | 40,6 | 2 | 225 | 160 | 385 | 58,9 | 4 P | 32 | 130 | 3/7/10/80 |
| | | | | U | 3,5 | – | 3,5 | 0,5 | | | | |

EP 0 382 054 B1

Tabelle 3 (Forts.)

Feste Farbmittelzubereitungen

| Vergleichs-beispiel Nr. | Farbmittel | | Dispergiermittel/Hilfsmittel | Menge vor der Mahlung [g] | nach der Mahlung [g] | zus. [g] | [%] | Mahldauer und -art [h] | Feststoff-gehalt der Dispersion vor Sprüh-trocknung [%] | Tempera-tur bei Sprüh-trocknung [C°] | Schleuder-wert |
|---|---|---|---|---|---|---|---|---|---|---|---|
| | | Menge [g] | [%] | | | | | | | | |
| 38 | J | 300 | 40 | Y | 200 | 250 | 450 | 60 | 4xS | 25 | 100 | 2/12/25/61 |
| 39 | K | 200 | 40 | Y | 150 | 150 | 300 | 60 | 3 P | 30 | 100 | 4/13/31/52 |
| 40 | F | 190 | 36,5 | Y | 60 | 270 | 330 | 63,5 | 6 P | 17 | 130 | 1/6/20/73 |
| 41 | G | 265 | 40,6 | X | 190 | 135 | 325 | 49,7 | 4 P | 32 | 130 | 1/4/17/78 |
| | | | | Y | 35 | 25 | 60 | 9,2 | | | | |
| | | | | U | 35 | – | 35 | 0,5 | | | | |
| 42 | H | 230 | 46 | X | 195 | 195 | 195 | 39 | 3xS | 20 | 130 | 6/16/32/46 |
| | | | | Y | 75 | 75 | 75 | 15 | | | | |

Bedeutung der Abkürzungen in Tabelle 2 und 3

Bei den eingesetzten Farbmitteln handelt es sich um folgende im Colour Index verzeichnete Farbstoffe:
A = Disperse Blue 60, C.I. 61100 (Anthrachinonfarbstoff)
B = Disperse Red 91 (Anthrachinonfarbstoff)

14

C = Disperse Yellow 64, C.I. 47023 (Oxichinophthalonfarbstoff)
D = Disperse Blue 79, C.I. 11345 (Azofarbstoff)
E = Fluorescent Brightening Agent 199 (Terephtahalsäurederivat)
F = Vat Blue 4, C.I. 69800 (Anthrachinonfarbstoff)
G = Vat Blue 6, C.I. 69825 (Anthrachinonfarbstoff)
H = Vat Green 1, C.I. 59825 (Anthrachinonfarbstoff)
J = Disperse Blue 330 (Azofarbstoff)
K = Disperse Red 167:1 (Azofarbstoff)

Folgende Hilfsmittel wurden mitverwendet:

L = Glycerin (als Wasserrückhaltemittel)
M = 1,2-Benzisothiazolin-3-on (als Biozid) in Form einer 9,5 %igen Lösung in Wasser/Propylenglykol
N = Sorbit (als Wasserrückhaltemittel)
Q = Propylenglykol (als Wasserrückhaltemittel)
R = Ethylenglykol (als Wasserrückhaltemittel)
T = Triethanolamin (als pH-Regulator)
U = Natriumsalz des Di-$C_9$-$C_{11}$-alkylsulfimids (als Netzmittel)

Folgende Mahlungsarten wurden benutzt:

P = Perlmahlung
S = Sandmahlung

Für die Vergleichsbeispiele wurde folgende handelsübliche Dispergiermittel verwendet:

X = Naphthalinsulfonsäure-Formaldehyd-Kondensat (in Form des Natrium-Salzes)
Y = Ligninsulfonat (in Form des Natrium-Salzes)

**Patentansprüche**

**Patentansprüche für folgende Vertragsstaaten : AT, BE, CH, DE, DK, FR, GB, IT, LI, NL, SE**

1. Chemisch modifizierte Proteine, erhältlich durch Umsetzung von Casein mit 0,5 bis 50 Gew.-% einer nicht aromatischen Mono- oder Disulfonsäure, die in ihrem organischen Rest eine oder mehrere mit Nucleophilen reagierende Gruppen oder Gruppierungen enthält, in wäßrigem Medium bei einem pH-Wert von 6 bis 14.

2. Chemisch modifizierte Proteine nach Anspruch 1, erhältlich durch Umsetzung mit 1 bis 20 Gew.-% einer nicht aromatischen Mono- oder Disulfonsäure, die in ihrem organischen Rest eine oder mehrere mit Nucleophilen reagierende Gruppen oder Gruppierungen enthält.

3. Chemisch modifizierte Proteine nach Anspruch 1 oder 2, erhältlich durch Umsetzung bei einem pH-Wert von 7 bis 12.

4. Verfahren zur Herstellung chemisch modifizierter Proteine gemäß den Ansprüchen 1 bis 3, dadurch gekennzeichnet, daß man Casein mit 0,5 bis 50 Gew.-% einer nicht aromatischen Mono- oder Disulfonsäure, die in ihrem organischen Rest eine oder mehrere mit Nucleophilen reagierende Gruppen oder Gruppierungen enthält, in wäßrigem Medium bei einem pH-Wert von 6 bis 14 umsetzt.

5. Verwendung von chemisch modifizierten Proteinen gemäß den Ansprüchen 1 bis 3 als Dispergiermittel in Farbmittelzubereitungen.

6. Feste wasserfreie oder flüssige wäßrige Farbmittelzubereitungen, enthaltend als Dispergiermittel ein chemisch modifiziertes Protein gemäß den Ansprüchen 1 bis 3.

7. Feste wasserfreie Farbmittelzubereitungen nach Anspruch 6, enthaltend 0,001 bis 10 Gew.-Teile des Dispergiermittels pro Gew.-Teil des Farbmittels.

8. Flüssige wäßrige Farbmittelzubereitungen nach Anspruch 6, enthaltend 0,001 bis 2 Gew.-Teile des Dispergiermittels pro Gew.-Teil des Farbmittels.

9. Farbmittelzubereitungen nach den Ansprüchen 6 bis 8, enthaltend als zusätzliche Dispergiermittel wasserlösliche Salze von Copolymerisaten aus olefinisch ungesättigten Carbonsäuren und/oder Carbonsäureanhydriden einerseits und wasserunlöslichen Monomeren andererseits.

EP 0 382 054 B1

**Patentansprüche für folgenden Vertragsstaat : ES**

**1.** Verfahren zur Herstellung chemisch modifizierter Proteine, dadurch gekennzeichnet, daß man Casein mit 0,5 bis 50 Gew.-% einer nicht aromatischen Mono- oder Disulfonsäure, die in ihrem organischen Rest eine oder mehrere mit Nucleophilen reagierende Gruppen oder Gruppierungen enthält, in wäßrigem Medium bei einem pH-Wert von 6 bis 14 umsetzt.

**2.** Verfahren nach Anspruch 1, dadurch gekennzeichnet, daß man mit 1 bis 20 Gew.-% einer nicht aromatischen Mono- oder Disulfonsäure umsetzt, die in ihrem organischen Rest eine oder mehrere mit Nucleophilen reagierende Gruppen oder Gruppierungen enthält.

**3.** Verfahren nach Anspruch 1 oder 2, dadurch gekennzeichnet, daß man bei einem pH-Wert von 7 bis 12 umsetzt.

**4.** Verfahren zur Herstellung von festen wasserfreien oder flüssigen wäßrigen Farbmittelzubereitungen, dadurch gekennzeichnet, daß man das Farbmittel und sonstige hierbei üblichen Bestandteile mit einem chemisch modifizierten Protein gemäß den Ansprüchen 1 bis 3 als Dispergiermittel mischt.

**5.** Verfahren zur Herstellung von festen wasserfreien Farbmittelzubereitungen nach Anspruch 4, dadurch gekennzeichnet, daß man 0,001 bis 10 Gew.-% Teile des Dispergiermittels pro Gew.-Teil des Farbmittels verwendet.

**6.** Verfahren zur Herstellung von flüssigen wäßrigen Farbmittelzubereitungen nach Anspruch 4, dadurch gekennzeichnet, daß man 0,001 bis 2 Gew.-Teile des Dispergiermittels pro Gew.-Teil des Farbmittels verwendet.

**7.** Verfahren nach den Ansprüchen 4 bis 6, dadurch gekennzeichnet, daß man als zusätzliches Dispergiermittel wasserlösliche Salze von Copolymerisaten aus olefinisch ungesättigten Carbonsäuren und/oder Carbonsäureanhydriden einerseits und wasserunlöslichen Monomeren andererseits verwendet.

**Claims**
**Claims for the following Contracting States : AT, BE, CH, DE, DK, FR, GB, IT, LI, NL, SE**

**1.** A chemically modified protein obtainable by reacting casein with 0.5 to 50% by weight of a non-aromatic mono- or disulfonic acid which contains in its organic radical one or more groups or groupings which react with nucleophiles, in an aqueous medium at a pH of from 6 to 14.

**2.** A chemically modified protein as claimed in claim 1, obtainable by reaction with 1 to 20% by weight of a non-aromatic mono- or disulfonic acid which contains in its organic radical one or more groups or groupings which react with nucleophiles.

**3.** A chemically modified protein as claimed in claim 1 or 2, obtainable by reaction at a pH of from 7 to 12.

**4.** A process for the preparation of a chemically modified protein as claimed in any of claims 1 to 3, which comprises reacting casein with 0.5 to 50% by weight of a non-aromatic mono- or disulfonic acid which contains in its organic radical one or more groups or groupings which react with nucleophiles, in an aqueous medium at a pH of from 6 to 14.

**5.** The use of a chemically modified protein as claimed in any of claims 1 to 3 as dispersant in a colorant formulation.

**6.** A solid anhydrous or liquid aqueous colorant formulation containing as dispersant a chemically modified protein as claimed in any of claims 1 to 3.

**7.** A solid anhydrous colorant formulation as claimed in claim 6, containing 0.001 to 10 parts by weight of the dispersant per part by weight of the colorant.

8. A liquid aqueous colorant formulation as claimed in claim 6, containing 0.001 to 2 parts by weight of the dispersant per part by weight of the colorant.

9. A colorant formulation as claimed in any of claims 6 to 8, containing as additional dispersants water-soluble salts of copolymers composed of olefinically unsaturated carboxylic acids, carboxylic anhydrides or mixtures thereof on the one hand, and of water-insoluble monomers on the other hand.

**Claims for the following Contracting State : ES**

1. A process for the preparation of a chemically modified protein, which comprises reacting casein with 0.5 to 50% by weight of a non-aromatic mono- or disulfonic acid which contains in its organic radical one or more groups or groupings which react with nucleophiles, in an aqueous medium at a pH of from 6 to 14.

2. A process as claimed in claim 1, wherein the reaction is carried out with 1 to 20% by weight of a non-aromatic mono- or disulfonic acid which contains in its organic radical one or more groups or groupings which react with nucleophiles.

3. A process as claimed in claim 1 or 2, wherein the reaction is carried out at a pH of from 7 to 12.

4. A process for the preparation of a solid anhydrous or liquid aqueous colorant formulation, which comprises mixing the colorant and other conventional constituents with a chemically modified protein as claimed in any of claims 1 to 3 as dispersant.

5. A process for the preparation of a solid anhydrous colorant formulation as claimed in claim 4, wherein 0.001 to 10 parts by weight of the dispersant are used per part by weight of the colorant.

6. A process for the preparation of a liquid aqueous colorant formulation as claimed in claim 4, wherein 0.001 to 2 parts by weight of the dispersant are used per part by weight of the colorant.

7. A process as claimed in any of claims 4 to 6, wherein water-soluble salts of copolymers composed of olefinically unsaturated carboxylic acids, carboxylic anhydrides or mixtures thereof on the one hand, and of water-insoluble monomers on the other hand, are used as additional dispersant.

**Revendications**
**Revendications pour les Etats contractants suivants : AT, BE, CH, DE, DK, FR, GB, IT, LI, NL, SE**

1. Protéines chimiquement modifiées, que l'on peut obtenir par la réaction de la caséine avec 0,5 à 50% en poids d'un acide mono- ou disulfonique, non aromatique, qui contient, dans son reste organique, un ou plusieurs radicaux ou groupements réagissant avec des nucléophiles, en milieu aqueux, à un pH de 6 à 14.

2. Protéines chimiquement modifiées suivant la revendication 1, que l'on peut obtenir par la réaction de 1 à 20% en poids d'un acide mono- ou disulfonique, non aromatique, qui contient, dans son reste organique, un ou plusieurs groupements ou radicaux réagissant avec des nucléophiles.

3. Protéines chimiquement modifiées suivant la revendication 1 ou 2, que l'on peut obtenir par réaction à un pH de 7 à 12.

4. Procédé de préparation de protéines chimiquement modifiées suivant les revendications 1 à 3, caractérisé en ce que l'on fait réagir la caséine avec 0,5 à 50% en poids d'un acide mono- ou disulfonique, non aromatique, qui contient, dans son reste organique, un ou plusieurs radicaux ou groupements réagissant avec des nucléophiles, en milieu aqueux et à une valeur de pH de 6 à 14.

5. Utilisation de protéines chimiquement modifiées suivant les revendications 1 à 3 à titre d'agents dispersifs dans des préparations de colorants.

**6.** Préparations de colorants solides, dépourvues d'eau, ou aqueuses et liquides, contenant, à titre d'agent dispersif, une protéine chimiquement modifiée suivant les revendications 1 à 3.

**7.** Préparations de colorants solides, dépourvues d'eau, suivant la revendication 6, contenant 0,001 à 10 parties en poids de l'agent dispersif par partie en poids du colorant.

**8.** Préparations de colorants aqueuses et liquides suivant la revendication 6, contenant de 0,001 à 2 parties en poids de l'agent dispersif par partie en poids du colorant.

**9.** Préparations de colorants suivant les revendications 6 à 8, contenant, à titre d'agents dispersifs supplémentaires, des sels solubles dans l'eau de copolymères d'acides carboxyliques oléfiniquement insaturés et/ou d'anhydrides d'acides carboxyliques oléfiniquement insaturés, d'une part et des monomères insolubles dans l'eau, d'autre part.

**Revendications pour l'Etat contractant suivant : ES**

**1.** Procédé de préparation de protéines chimiquement modifiées, caractérisé en ce que l'on fait réagir la caséine avec 0,5 à 50% en poids d'un acide mono- ou disulfonique, non aromatique, qui contient, dans son reste organique, un ou plusieurs radicaux ou groupements réagissant avec des nucléophiles, en milieu aqueux et à une valeur de pH de 6 à 14.

**2.** Procédé suivant la revendication 1, caractérisé en ce que l'on opère la réaction avec 1 à 20% en poids d'un acide mono- ou disulfonique, non aromatique, qui contient, dans son reste organique, un ou plusieurs radicaux ou groupements réagissant avec des nucléophiles.

**3.** Procédé suivant la revendication 1 ou 2, caractérisé en ce que l'on opère la réaction à un pH de 7 à 12.

**4.** Procédé de fabrication de préparations de colorants solides, dépourvues d'eau, ou aqueuses et liquides, caractérisé en ce que l'on mélange le colorant et certains constituants habituels à cette fin avec une protéine chimiquement modifiée suivant l'une quelconque des revendications 1 à 3, à titre d'agent dispersif.

**5.** Procédé de fabrication de préparations de colorants solides, dépourvues d'eau, suivant la revendication 4, caractérisé en ce que l'on utilise de 0,001 à 10% en poids de l'agent dispersif par partie en poids du colorant.

**6.** Procédé de fabrication de préparations de colorants aqueuses et liquides suivant la revendication 4, caractérisé en ce que l'on utilise de 0,001 à 2 parties en poids de l'agent dispersif par partie en poids du colorant.

**7.** Procédé suivant l'une quelconque des revendications 4 à 6, caractérisé en ce que l'on utilise, à titre d'agent dispersif supplémentaire, des sels solubles dans l'eau de copolymères d'acides carboxyliques oléfiniquement insaturés et/ou d'anhydrides d'acides carboxyliques oléfiniquement insaturés d'une part et des monomères insolubles dans l'eau d'autre part.